# FASCICULE DE BREVET EUROPEEN

(11) **EP 1 108 043 B1**
(45) Date de publication et mention de la délivrance du brevet: **31.03.2004**
(21) Numéro de dépôt: 99940267.0
(22) Date de dépôt: 01.09.1999
(51) Int. Cl.: C12N 15/81, C12N 1/19

(54) **PROCEDE DE TRANSFORMATION NON-HOMOLOGUE DE YARROWIA LIPOLYTICA**
NICHT-HOMOLOGUES YARROWIA LIPOLYTICA TRANSFORMATIONSVERFAHREN
METHOD FOR NON-HOMOLOGOUS TRANSFORMATION OF YARROWIA LIPOLYTICA

(30) Priorité: 01.09.1998 FR 9810900
(43) Date de publication de la demande: 20.06.2001
(73) Titulaire: INSTITUT NATIONAL DE LA RECHERCHE AGRONOMIQUE (INRA), 75338 Paris Cédex 07 (FR); CENTRE NATIONAL DE LA RECHERCHE SCIENTIFIQUE (CNRS), 75794 Paris Cedex 16 (FR)
(72) Inventeur: NICAUD, Jean-Marc, F-78190 Trappes (FR); GAILLARDIN, Claude, F-78000 Versailles (FR); PIGNEDE, Georges, F-92270 Bois Colombes (FR)
(74) Mandataire: Vialle-Presles, Marie José
(86) Numéro de dépôt international: PCT/FR1999/002079
(87) Numéro de publication internationale: WO 2000/012729

(56) Documents cités:
- EP-A- 0 138 508
- EP-A- 0 220 864
- FR-A- 2 665 908
- C. NEUV GLISE ET AL.: "A shuttle mutagenesis system for tagging genes in the yeast Yarrowia lipolytica" GENE, vol. 213, juin 1998 (1998-06), pages 37-46, XP004125001 AMSTERDAM NL
- MARIE-TH R SE LE DALL ET AL.: "Multiple-copy integration on the yeast Yarrowia lipolytica" CURRENT GENETICS, vol. 26, 1994, pages 38-44, XP002040754
- NATASCHA SCHMID-BERGER ET AL.: "Ylt1, a highly repetitive retrotransposon in the genome of the dimorphic fungus Yarrowia lipolytica" JOURNAL OF BACTERIOLOGY, vol. 176, no. 9, mai 1994 (1994-05), pages 2477-2482, XP002103773 cité dans la demande

## Description

L'invention est relative à des outils d'expression de gènes hétérologues chez *Yarrowia lipolytica*.

La levure *Y. lipolytica* est de plus en plus employée comme hôte d'expression de gènes d'intérêt ; on utilise en particulier dans ce cadre, des vecteurs dits « intégratifs », qui permettent l'insertion d'un segment d'ADN portant le gène d'intérêt dans l'ADN chromosomique. Par exemple, la Demande EP 138 508 décrit la transformation de *Yarrowia lipolytica* à l'aide de vecteurs capables de s'intégrer dans l'ADN chromosomique par recombinaison de séquences de *Y. lipolytica* portées par lesdits vecteurs, avec les séquences homologues présentes sur l'ADN chromosomique de la cellule hôte.

Parmi les séquences de *Yarrowia lipolytica* utilisées pour la construction de vecteurs intégratifs, on citera notamment les séquences dénommées : « séquences zéta », qui correspondent aux LRT (longues répétitions terminales) du rétrotransposon Ylt1 de *Yarrowia lipolytica* ; ces séquences ont été décrites par SCHMID-BERGER et al. [J. Bacteriol., 2477-2482 (1994)] qui indiquent qu'elles sont présentes en un grand nombre de copies (environ 35 copies du rétrotransposon complet, et environ 30 copies de la séquence zéta isolée) dans l'ADN chromosomique de certaines souches de *Yarrowia lipolytica*. Lorsqu'un vecteur contenant un insert flanqué de séquences zéta est utilisé pour transformer l'une de ces souches de *Yarrowia,* l'ADN de l'insert s'intègre par recombinaison homologue avec des séquences zéta de l'ADN chromosomique. On obtient de la sorte des cellules de *Yarrowia* transformées contenant plusieurs copies d'une séquence hétérologue intégrées en tandem au niveau de sites zéta chromosomiques.

Les Inventeurs ont maintenant constaté que de manière surprenante, lorsque des vecteurs portant des inserts flanqués de séquences zéta étaient utilisés pour transformer des cellules de *Yarrowia* dépourvues de ces séquences, l'ADN de l'insert s'intégrait cependant dans l'ADN chromosomique, sous la forme de plusieurs copies dispersées dans le génome.

La présente invention a pour objet un procédé d'intégration d'un gène d'intérêt dans le génome d'une souche de *Yarrowia,* à l'aide d'un vecteur recombinant portant un insert flanqué de séquences zéta et comprenant ledit gène d'intérêt, lequel procédé est caractérisé en ce l'on met en oeuvre ledit vecteur recombinant pour transformer une souche de *Yarrowia* dont le génome est dépourvu de séquences zéta.

Des souches de *Yarrowia lipolytica* dépourvues de séquences zéta, utilisables pour la mise en oeuvre du procédé conforme à l'invention sont par exemple les souches dérivées de la souche W29 (ATCC 20460 ou CLIB 89, MatA), telles que W29ura3-302 (CLIB 141, MatA Ura3-302), PO1a (CLIB 140, MatA Ura3-302, Leu2-270) et PO1d (CLIB 139, MatA Ura3-302, Leu2-270, xpr2-322), décrites par BARTH et GAILLARDIN [The dimorphic fungus *Yarrowia lipolytica*. In: *Non conventional yeasts in biotechnology* (Wolf K. Ed.). Springer-Verlag, Berlin pp 313-388 (1996)], et que l'on peut se procurer auprès de la CLIB, (Collection de Levures d'intérêt Biotechnologique, INRA, Centre de Grignon, BP01, 78850 Thiberval-Grignon). D'autres souches utilisables peuvent facilement être sélectionnées sur la base de l'absence de signaux d'hybridation avec des sondes d'acide nucléique dérivées des séquences zéta.

Selon un mode de mise en oeuvre préféré du procédé conforme à l'invention, l'insert comprenant le gène d'intérêt comprend en outre des séquences permettant le contrôle de l'expression dudit gène et/ou au moins un marqueur de sélection des transformants.

Le marqueur de sélection est constitué par un gène dont l'expression permet la sélection des transformants. Il peut s'agir par exemple d'un gène de résistance à un antibiotique, ou d'un mutant défectif d'un gène nécessaire à la croissance de la levure, tel que URA3, LEU2, etc. Avantageusement, on choisira un marqueur de sélection nécessitant d'être présent en plusieurs copies pour être fonctionnel, ce qui permet de sélectionner les transformants ayant intégré plusieurs copies du gène d'intérêt. Par exemple, on peut utiliser comme marqueur de sélection un marqueur URA3 défectif, qui dérive du gène *URA3* de *Y. lipolytica* permettant la complémentation de l'auxotrophie pour l'uracile, tel que les marqueurs URA3d décrits par LE DALL et al. [Curr. Genet., 26, 38-44 (1994)].

Les séquences de contrôle de l'expression sont notamment des séquences promoteur et terminateur actives chez *Yarrowia.* On peut utiliser un promoteur inductible ou constitutif.

Très avantageusement, on peut également utiliser en tant que séquences de contrôle, le promoteur du gène de l'acyl CoA oxydase ACO2 de *Yarrowia lipolytica,* [LE CLAINCHE, Thèse de doctorat de l'Institut National Agronomique Paris Grignon, soutenue le 2 juillet 1997], ou le promoteur et/ou le terminateur du gène *LIP2* de la lipase extracellulaire acidorésistante de *Yarrowia lipolytica*, décrit dans la Demande Française au nom de LABORATOIRES MAYOLY-SPINDLER, intitulée : « CLONAGE ET EXPRESSION DE LIPASES EXTRACELLULAIRES ACIDORESISTANTES DE LEVURES », déposée le même jour que la présente Demande.

Les promoteurs *ACO2* et *LIP2* sont tous deux inductibles par les triglycérides et les acides gras.

D'autres marqueurs de sélection et séquences de contrôle utilisables pour la mise en oeuvre de la présente invention sont par exemple ceux cités par BARTH et GAILLARDIN (publication précitée).

Lorsque le produit du gène d'intérêt est destiné à être sécrété par la cellule-hôte, ledit insert comprend en outre des signaux de contrôle de la sécrétion dudit produit. On peut utiliser dans ce but des séquences signal fonctionnelles chez *Yarrowia lipolytica*, par exemple tout ou partie de la séquence prépro du gène *LIP2* décrit dans la Demande Française au nom de LABORATOIRES MAYOLY-SPINDLER, mentionnée ci-dessus.

La présente invention a également pour objet des cellules de *Yarrowia* transformées susceptibles d'être obtenues par le procédé conforme à l'invention. Avantageusement, ces cellules transformées comprennent au moins 2 copies de l'insert flanqué de séquences zéta, comprenant le gène d'intérêt, intégrées dans leur génome de manière dispersée.

La présente invention sera mieux comprise à l'aide du complément de description qui va suivre, qui se réfère à des exemples non-limitatifs d'obtention de souches transformées de *Yarrowia lipolytica* conformes à l'invention.

### EXEMPLE 1 : CONSTRUCTION DE VECTEURS COMPRENANT UNE SEQUENCE ZETA

Ces vecteurs sont obtenus à partir du vecteur pINA1067, dérivé d'un vecteur de type pINA970 décrit par BARTH et GAILLARDIN (publication précitée, Fig. 6 ), par excision de la portion de séquence comprise entre le promoteur et le terminateur XPR2, qui a été remplacée par un lieur contenant les sites de restriction SrfI et BglII. pINA1067 porte le marqueur URA3 défectif *ura3d4* décrit par LE DALL et al. (publication précitée), ainsi qu'un marqueur de résistance à la tétracycline.

Le plasmide pHSS6 portant une origine de réplication pour *E. coli* et un gène de résistance à la kanamycine est linéarisé par restriction avec NotI et introduit au site NotI de pINA1067. Le mélange de ligation est utilisé pour transformer *E. coli*. Les transformants ayant intégré le plasmide sont sélectionnés sur tétracycline (6 mg/l) et kanamycine (40 mg/l).

Le vecteur résultant, dénommé JMP1, est coupé par HindIII et EcoRI, ce qui élimine le fragment portant les séquences promoteur et terminateur de XPR2 et le marqueur de résistance à la tétracycline ; ce fragment est remplacé par un lieur multisite qui contient les sites de restriction HindIII, ClaI, MluI, HpaI, BamHI et EcoRI.

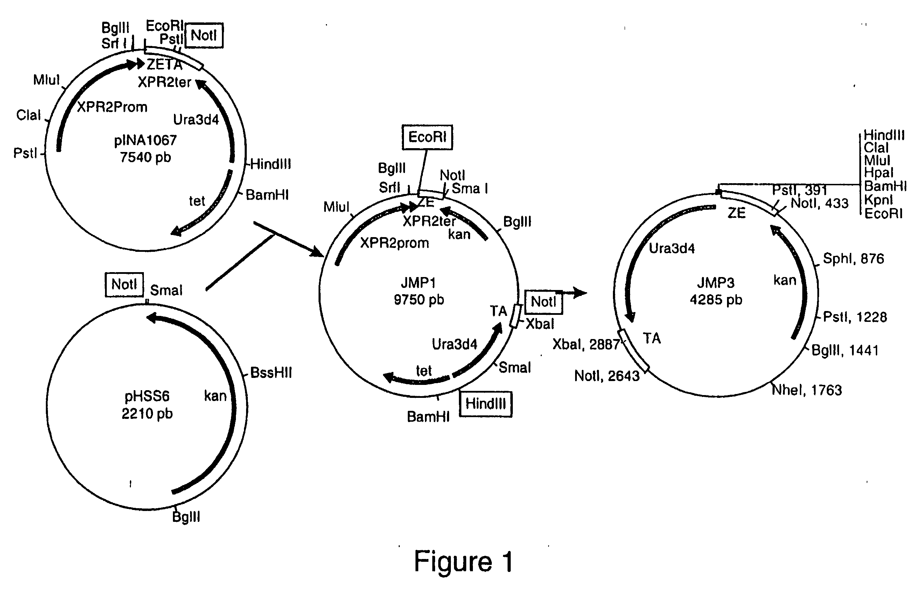

Le vecteur résultant est dénommé JMP3. Les différentes étapes de la construction de JMP3 sont schématisées sur la figure 1.

Les vecteurs d'autoclonage JMP3 et JMP5 s'intègrent dans le génome après coupure par l'enzyme de restriction Not*I*. Cette restriction permet l'élimination de la partie bactérienne (origine de réplication de *E. coli* et du marqueur de résistance KanR).

### EXEMPLE 2 : CONSTRUCTION DE VECTEURS D'EXPRESSION

Des vecteurs d'expression comprenant une séquence codant pour la lipase extracellulaire acidorésistante de *Yarrowia lipolytica*, décrite dans la Demande Française au nom des LABORATOIRES MAYOLY-SPINDLER, mentionnée ci-dessus, sous contrôle du promoteur ACO2 (vecteur JMP6) ou de son propre promoteur (vecteur JMP10) ont été construits.

### Vecteur JMP6 :

Le promoteur du gène de l'acyl CoA oxidase ACO2 a été amplifié par PCR à partir de l'ADN génomique total de *Y. lipolytica,* avec les oligonucléotides Aco2P1 et Aco2P2 qui contiennent respectivement un site ClaI et un site HindIII.

Aco2P1 :
5'
GCG ATCGAT CATACTGTTACACTGCTCCG
3'

Aco2P2 :
5'
GTGGGATCCGAAAGCTTCATGGCGTCGTTGCTTGTGTGATTTTTGAGG
3'

Le fragment de PCR de 2168 pb a été cloné au site EcoRV du vecteur BLUESCRIPT KS+. Le plasmide résultant est appelé KS-ACO2prom. Ce vecteur a été digéré par ClaI et partiellement par HindIII pour obtenir un fragment de 2155pb contenant le promoteur ACO2 complet.

D'autre part un fragment HindIII-EcoRI de 1134pb contenant la séquence codant pour la prolipase LIP2, ainsi que le terminateur de transcription, a été isolé du plasmide dénommé pKS+-LIP2W29a. Les deux fragments purifiés sont fusionnés et insérés aux sites ClaI-EcoRI du vecteur JMP3. Le plasmide résultant est appelé JMP6.

### Vecteur JMP10 :

Un fragment NdeI*-HindIII de 2030 pb contenant le promoteur du gène de la lipase de *Y. lipolytica* a été isolé à partir du plasmide dénommé pKS+-LIP2prom. Pour cela le plasmide pKS+-LIP2prom a été digéré par NdeI, puis traité à la T4 DNA polymérase pour rendre le site NdeI franc (NdeI*). Après inactivation de l'enzyme NdeI et de la polymérase, l'ADN a été coupé par HindIII. Le fragment NdeI*-HindIII de 2030 pb a été ligaturé avec le fragment HindIII-EcoRI de 1134 pb portant le gène de la lipase et son terminateur, et le produit de ligation inséré aux sites HpaI-EcoRI du vecteur JMP3. Le plasmide résultant est appelé JMP10.

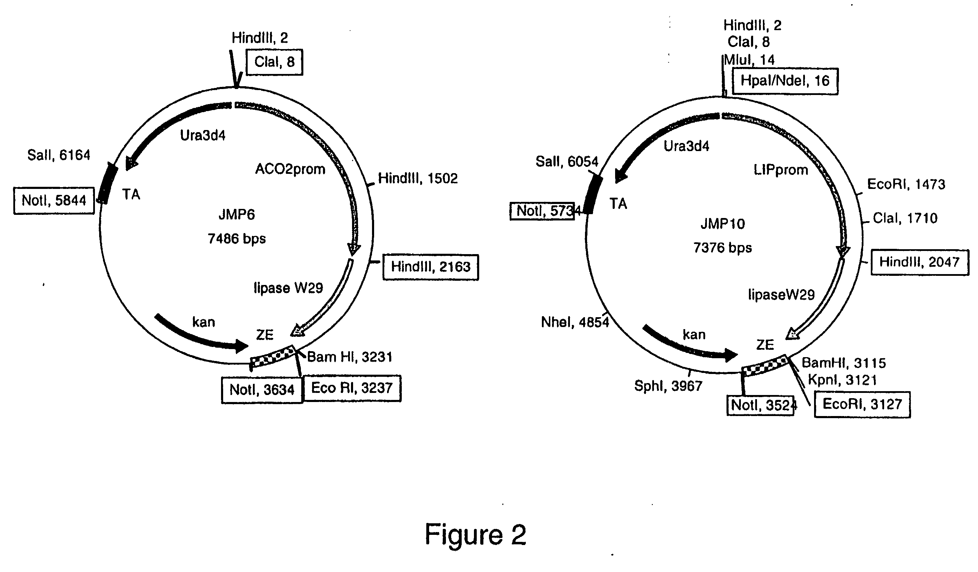

La figure 2 représente les plasmides JMP6 et JMP10.

### EXEMPLE 3 : OBTENTION DE TRANSFORMANTS DE Y. LIPOLYTICA.

Les souches de *Y. lipolytica* suivantes, présentant une délétion du gène *URA3*, ont été utilisées :
- PO1d : (CLIB 139, MatA Ura3-302, Leu2-270, xpr2-322), dépourvue de séquences zéta ;
- E150 : (CLIB 122, MatA Ura3-302, Leu2-270, xpr2-322, his-1) possédant plusieurs copies de séquences zéta.

Ces deux souches, décrites par BARTH et GAILLARDIN (publication précitée) sont disponibles auprès de la CLIB.

Les plasmides JMP6 et JMP10, préalablement linéarisés par NotI, ce qui permet d'éliminer les séquences provenant du plasmide pHSS6, sont introduits dans les cellules de *Yarrowia* par transformation à l'acétate de lithium, selon le protocole décrit par BARTH et GAILLARDIN.

On obtient habituellement 20 à 50 transformants/µg de DNA avec la souche PO1d et 100 à 200 transformants/µg de DNA avec la souche E150.

Les différents transformants seront désignés ci-après selon la nomenclature suivante : nom de la souche - numéro du plasmide - numéro du transformant. Par exemple ; PO1d-6-15 = souche PO1d, plasmide JMP6, transformant numéro 15.

La structure des transformants a été étudiée par transfert de Southern. L'ADN génomique total de la souche d'origine (T) et des différents transformants est digéré par HindIII dans le cas de PO1d et par BamHI (pour les transformants JMP6) ou EcoRI (pour les transformants JMP10)dans le cas de E150.

Les sondes utilisées sont dérivées des séquences zéta, de la séquence du promoteur ACO2, et de la séquence codant pour la lipase LIP2.

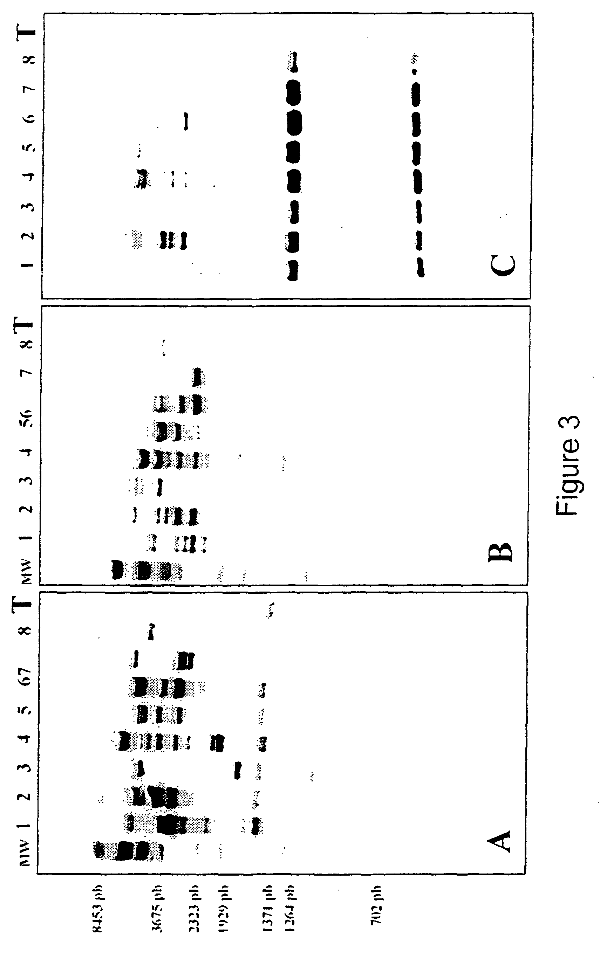

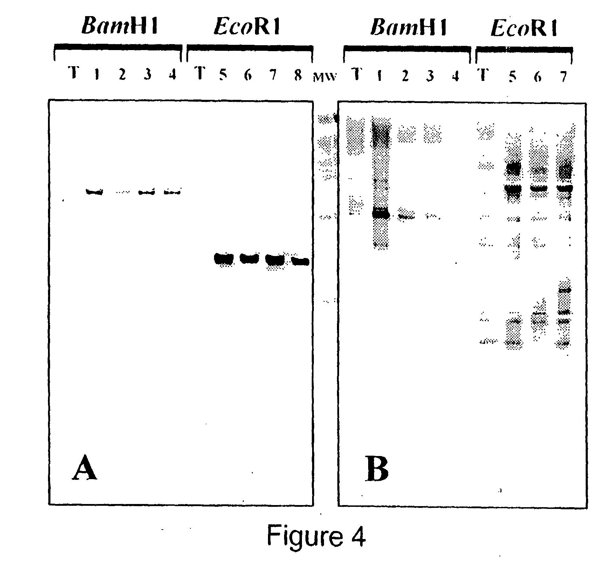

Les résultats obtenus sont illustrés par la figure 3 pour les transformants PO1d, et par la figure 4 pour les tranformants E150.

### Transformants PO1d (JMP6)

La figure 3A représente les résultats observés avec la sonde lipase ; la figure 3B représente les résultats observés avec la sonde ZETA ; la figure 3C représente les résultats observés avec la sonde ACO2.

Dans la souche PO1d (ligne T) on observe une bande de 1,4 kb avec la sonde lipase, aucune bande avec la sonde zéta (absence de site zéta dans cette souche) et une bande de 1,1 kb avec la sonde ACO2. Dans le cas des transformants, on observe avec les sondes lipase et zéta de nombreuses bandes de taille variable, correspondant au nombre de copies et montrant qu'elles sont dispersées dans le génome.

On observe donc dans le cas des souches transformantes conformes à l'invention, une intégration non homologue, en multicopie et dispersée.

### Transformants E150 (JMP6 et JMP10).

La figure 4A représente les résultats observés avec la sonde lipase + la sonde ACO2 ; la figure 4B représente les résultats observés avec la sonde ZETA.

Sur la Figure 4A, on observe chez les transformants, une amplification des séquences du vecteur, révélée par l'intensité de la bande BamHI de 2,9 kb (promoteur ACO2 + gène de la lipase) ou de la bande EcoRI de 1,6 kb (gène de la lipase), comparée avec celle de la bande génomique BamHI de 2,6 kb qui correspond au promoteur *ACO2* génomique, ou celle de la bande génomique EcoRI d'environ 6 kb, qui correspond au gène de la lipase.

Sur la figure 4B, on observe dans la souche E150, et les transformants plusieurs sites zéta révélés par de nombreuses bandes dans le profil de restriction EcoRI. L'intégration en tandem est révélée par l'intensité de la bande BamHI de 2,5 kb, et la bande EcoRI de 3,7 kb, correspondant aux fragments attendus si les séquences des vecteurs sont intégrées en tandem.

Chez le transformant 6 on remarque en outre la disparition d'une bande zéta indiquant l'intégration dans ce locus.

On observe donc, dans le cas des transformants de la souche E150, une intégration en tandem par intégration homologue à un site zéta.

### Sécrétion de lipase par les souches transformées.

La production de lipase par les souches transformées a été testée sur différents milieux. On observe, aussi bien dans le cas des souches conformes à l'invention dérivées de PO1d, que dans celui des souches de comparaison dérivées de E150, une sécrétion au moins dix à quinze fois supérieure à celle des souches non transformées.

## Revendications

1. Procédé d'intégration d'un gène d'intérêt dans le génome d'une souche de *Yarrowia*, à l'aide d'un vecteur recombinant portant un insert flanqué de séquences zéta et comprenant ledit gène d'intérêt, **caractérisé en ce** l'on met en oeuvre ledit vecteur recombinant pour transformer une souche de *Yarrowia* dont le génome est dépourvu de séquences zéta.

2. Procédé selon la revendication 1, **caractérisé en ce que** l'insert comprenant le gène d'intérêt comprend en outre des séquences permettant le contrôle de l'expression dudit gène et/ou au moins un marqueur de sélection des transformants.

3. Procédé selon la revendication 2, **caractérisé en ce que** ledit marqueur de sélection est un marqueur URA3 défectif.

4. Procédé selon une quelconque des revendications 2 ou 3, **caractérisé en ce qu'**au moins une des séquences permettant le contrôle de l'expression du gène d'intérêt est choisie parmi : le promoteur du gène de l'acyl CoA oxydase ACO2 de *Yarrowia lipolytica,* le promoteur du gène de la lipase extracellulaire acidorésistante de *Yarrowia lipolytica,* le terminateur du gène de la lipase extracellulaire acidorésistante de *Yarrowia lipolytica*.

5. Procédé selon une quelconque des revendications 2 à 4, **caractérisé en ce que** ladite cassette d'expression comprend en outre une séquence codant pour des signaux de contrôle de la sécrétion du produit dudit gène d'intérêt.

6. Procédé selon la revendication 5, **caractérisé en ce que** lesdits signaux de contrôle comprenent tout ou partie de la séquence prépro de la lipase extracellulaire acidorésistante de *Yarrowia lipolytica*.

7. Cellule de *Yarrowia* transformée, susceptible d'être obtenue par un procédé selon une quelconque des revendications 1 à 6.

8. Cellule transformée selon la revendication 7, **caractérisée en ce qu'**elle comprend au moins 2 copies des séquences dudit vecteur, intégrées dans son génome de manière dispersée.

## Patentansprüche

1. Verfahren zur Integration eines interessierenden Gens in das Genom eines Züchtungsstamms von *Yarrowia*, mit Hilfe eines rekombinanten Vektors, der einen auf beiden Seiten von Zeta-Sequenzen umgebenen Insert trägt und das interessierende Gen umfasst, **dadurch gekennzeichnet, dass** der rekombinante Vektor dazu verwendet wird, einen Züchtungsstamm von *Yarrowia* zu transformieren, dessen Genom keine Zeta-Sequenzen enthält.

2. Verfahren gemäß Anspruch 1, **dadurch gekennzeichnet, dass** der das interessierende Gen umfassende Insert außerdem Sequenzen, die die Kontrolle der Expression des Gens erlauben und/oder mindestens einen Selektionsmarker für die Transformanten umfasst.

3. Verfahren gemäß Anspruch 2, **dadurch gekennzeichnet, dass** der Selektionsmarker ein Marker durch fehlerhafte URA3 ist.

4. Verfahren gemäß einem der Ansprüche 2 oder 3, **dadurch gekennzeichnet, dass** mindestens eine der Sequenzen, die die Kontrolle der Expression des interessierenden Gens erlauben, ausgewählt ist aus: dem Promotor des Gens der Acyl - CoA - Oxidase ACO2 von *Yarrowia lipolytica*, dem Promotor des Gens der säureresistenten extrazellulären Lipase von *Yarrowia lipolytica*, dem Terminator des Gens der säureresistenten extrazellulären Lipase von *Yarrowia lipolytica*.

5. Verfahren gemäß einem der Ansprüche 2 bis 4, **dadurch gekennzeichnet, dass** die Expressionskassette außerdem eine Sequenz enthält, welche die Signale zur Kontrolle der Sekretion des Produktes des interessierenden Gens enthält.

6. Verfahren gemäß dem Anspruch 5, **dadurch gekennzeichnet, dass** die Kontrollsignale die ganze oder einen Teil der Präpro - Sequenz der säureresistenten extrazellulären Lipase von *Yarrowia lipolytica* umfassen.

7. Transformierte Zelle von *Yarrowia*, die dazu geeignet ist, mittels eines Verfahrens gemäß irgendeinem der Ansprüche 1 bis 6 erhalten zu werden.

8. Transformierte Zelle gemäß dem Anspruch 7, **dadurch gekennzeichnet, dass** sie mindestens 2 Kopien der Sequenzen des Vektors enthält, die auf verteilte Art in sein Genom integriert sind.

## Claims

1. Method for integrating a gene of interest into the genome of a strain of *Yarrowia*, using a recombinant vector bearing an insert flanked by zeta sequences and comprising said gene of interest, **characterized in that** said recombinant vector is used to transform a strain of *Yarrowia*, the genome of which lacks zeta sequences.

2. Method according to Claim 1, **characterized in that** the insert comprising the gene of interest also comprises sequences allowing the control of the expression of said gene and/or at least one marker for selection of the transformants.

3. Method according to Claim 2, **characterized in that** said selection marker is a defective URA3 marker.

4. Method according to either one of Claims 2 and 3, **characterized in that** at least one of the sequences allowing the control of the expression of the gene of interest is chosen from: the promoter of the acyl CoA oxidase gene AC02 of *Yarrowia lipolytica*, the promoter of the acid-resistant extracellular lipase gene of *Yarrowia lipolytica* and the terminator of the acid-resistant extracellular lipase gene of *Yarrowia lipolytica*.

5. Method according to any one of Claims 2 to 4, **characterized in that** said expression cassette also comprises a sequence encoding signals for controlling the secretion of the product of said gene of interest.

6. Method according to Claim 5, **characterized in that** said control signals comprise all or part of the prepro sequence of the acid-resistant extracellular lipase gene of *Yarrowia lipolytica*.

7. Transformed *Yarrowia* cell which can be obtained using a method according to any one of Claims 1 to 6.

8. Transformed cell according to Claim 7, **characterized in that** it comprises at least 2 copies of the sequences of said vector, integrated into its genome in a dispersed manner.
